# EUROPEAN PATENT APPLICATION

(11) **EP 3 716 278 A1**
(43) Date of publication of application: **30.09.2020**
(21) Application number: 19166267.5
(22) Date of filing: 29.03.2019
(51) Int. Cl.: G16H 20/30

(54) **SYSTEM AND METHOD FOR MOTOR/NEUROLOGICAL REHABILITATION**

(71) Applicant: Centrum Radosc MED Spolka z ograniczona Odpowiedzialnoscia Sp.k., 02-674 Warszawa (PL)
(72) Inventor: DMOWSKI, Andrzej, 04-691 Warszawa (PL)
(74) Representative: Marcinska-Porzuc, Aleksandra

(57) **Abstract**

The subject matter of the invention is a method for rehabilitation of individuals with motor/neurological disabilities and/or motor/intellectual disabilities using a holographic device, characterized in that an image displaying means (406, 506) of a holographic device (250) displays, during a single rehabilitation session, at least one personalized hologram that issues, in real time, via at least one signal generating means (408, 508) for generating a signal, a voice command and/or by a motion, a command to perform at least one rehabilitation activity by the patient before the holographic device (250), and at least one motion capturing means (410, 510) records the performance of said rehabilitation activity and communicates the footage of a rehabilitation session, via a footage communicating means (412, 512), to the central system (200) to verify the correctness of performing the rehabilitation activity, whereafter the central system (200) analyzes, in real time, the data obtained from the holographic device (250) and related to the performance, by the patient, of said rehabilitation activity and communicates, to the holographic device (250), commands, that are then communicated to the patient by the personalized hologram via at least one signal generating means (408, 508) for generating a signal as a voice command and/or by a motion, the personalized hologram being a three-dimensional image of an event, landscape, person, architecture, individuals in a group, that induces emotional associations for a patient. Also, the subject matter of the invention is a holographic device (250) and a system, and the use of the method, the system, and the holographic device (250) described in the present invention, for rehabilitation of individuals with motor/neurological disabilities and/or motor/intellectual disabilities.

## Description

### Field

This invention relates to a method and system for motor or neurological rehabilitation of individuals with motor/neurological or motor/intellectual disabilities, based on using, during rehabilitation, a holographic device communicating a personalized image, and the use of the device, method, and system disclosed in the present invention for motor or neurological rehabilitation of individuals with motor/neurological or motor/intellectual disabilities.

Motor/neurological or motor/intellectual disability handicaps social or psychological functioning of an individual and has attributes of suffering. Neurological or intellectual rehabilitation covers many different medical problems: conditions after skull/brain traumas, spinal cord traumas, it is recommended for patients suffering from progressive autoimmune diseases such that multiple sclerosis (SM) or amyutrophic lateral sclerosis (ALS), Parkinson's disease, diseases of peripheral nerves, or Alzheimer's disease, as well as children: autistic or with Down syndrome. For example, the Alzheimer's disease (AD) is the most commonly spread form of dementia among older persons, and is, probably, the fourth cause of deaths in developed countries. Cortical atrophy, atrophy of neurons, locally specific build up of amyloid, amyloid plaques (neuritic), and neurofibrillary tangles are the key features of the brain of an AD patient. It is believed, in science, that these changes are related to deterioration of cognitive functions that defines the AD clinically.

A significant issue with neurological dysfunctions is a high level of disability of patients suffering from these problems, progressing disease process and cognitive dysfunctions associating a motor disability.

### State of the Art

Various methods are known in the art. for rehabilitation of individuals who knowingly and wilfully do rehabilitation activities, as well as individuals who, although being passive in terms of performed actions, at least accept rehabilitation exercises.

Nowadays, one can observe using still newer and newer technological solutions in the rehabilitation process. One of them is a device Kinect that, due to virtual reality (VR), allows for interaction of a user with the console via limbs movements and gestures of the whole body. This gives an opportunity for rehabilitation of motor dysfunctions. In the Kinect device, a camera is used for reading more than 30 elements of the user's body, this making possible to collect data about motor capabilities of the patients and its later analysis in terms of the correctness of the movements of the upper limbs. These studies were carried out by L.R. Reitcher, M.H. Foreman, and N. Migorsky. Children take part in such rehabilitation procedures willingly, since it is a form of play and competition for them.

From PL229892 B a method is known for motor and educational rehabilitation of individuals with dysfunctions of upper limbs using a device having a display displaying a command to make movements, by the person being rehabilitated, with his/her hand in a space within frames of the device, in a given order, and then the correctness of performing the commands is controlled by means of sensors.

Also, a method is known in the art for rehabilitation of patients suffering from an injured muscle and/or nerve, the method being based on a measurement of patient's brain activity related to control of the injured muscle and/or nerve, and on a measurement of muscle activity of the injured muscle and/or nerve, in order to make a realistic visualization of every motion make with a dysfunctional body part, and then displaying a possible motion on a computer screen or another portable device. Such realistic visualization of a planned motion on the screen generates a feedback between the patient's brain, induced by visual stimuli, and the reaction, to this visualization, of the injured muscles.

Known, in the art, rehabilitation software associates the rehabilitation process with a play with an intention of encouraging the participants of the rehabilitation procedure to increase the efforts and engagement into the exercises. However, most of the virtual reality games are not designed for rehabilitation purposes for individuals with intellectual or neurological disabilities.

For example, this is a case of older individuals suffering from AD (Alzheimer's disease) who have already problems to find themselves in the real reality, and yet bigger problems in the virtual reality.

Rehabilitation of individuals with intellectual or neurological disabilities, including individuals with dementia, is extremely difficult, and sometimes practically impossible, because such patients do not want to do exercises, cooperate with rehabilitators, therapists, logopedists, pedagogs, and accept only very few people in any close relations.

Known, in the art, methods of rehabilitation make use, as instructors issuing commands for motor activities, avatars or holograms of graphically designed forms, however do not have the capability of reacting to the user's motion or emotions in real time. Additionally, often one needs portable devices put on the head, to make possible to receive the hologram.

### Summary of the invention

Therefore, the aim of the present invention is to provide a holographic device for motor/neurological rehabilitation, preferably for motor/neurological disabilities, and motor - general disabilities, motor-intellectual disabilities, including all types of oligophrenia of children and adults, of older individuals with syndroms of significant senile dementia, in particular with Alzheimer's disease, as well as a method for rehabilitation and a system of rehabilitation using a holographic device. Unexpectedly, the problem has been solved, to a significant extent, by the present invention.

The subject matter of the invention is a method for rehabilitation of individuals with motor/neurological disabilities and/or motor/intellectual disabilities, the method comprising:
- generating, in a central system, recommendations for a given rehabilitation session for a given patient identifier,
- parametrizing at least one personalized hologram, this being a three-dimensional image of an event, landscape, person, architecture, individuals in a group, inducing emotional associations for a patient, and, optionally, related to the history of the patient,
- sending, by the central system, at least one personalized hologram to a holographic device,
- generating, by image displaying means of a holographic device, a first personalized hologram,
- issuing to the patient, by the first personalized hologram, a command to perform rehabilitative actions by a motion and/or voice command,
- recording, by a motion capturing means and/or a footage communicating means, a fact of performing, by the patient, the rehabilitation activities, his/her emotional condition, and the course of the rehabilitation session,
- Establishing a communication with the receiving module of the central system,
- recording the generated images and the patient during given rehabilitation session,
- communicating, in real time, the footage of a given rehabilitation session to the central system in order to check the emotional and/or physical condition and/or to check the correctness of performing, by the patient, the rehabilitation activities.

Additionally, the method comprises:
- introducing a patient identifier into the central system,
- receiving, in real time, by the holographic device, information from the central system about the course of the rehabilitation session,
- verification, in real time, of the course of the rehabilitation session and the correctness of the performed rehabilitation activities.

In the case of a negative result of the verification of performing rehabilitation activities by the patient during a rehabilitation session, the method comprises receiving a signal from the central system about a possibility of repeating the rehabilitation activity that is repeated by the patient, and in the case the patient wishes not to repeat the rehabilitation activity, the central system investigates the emotional and/or physical condition of the patient, and basing on the read emotional and/or physical condition of the patient, communicates a signal to a holographic device, that, by means of a personalized hologram displayed by the image displaying means, issues, by means of a voice command, via a signal generation means, and/or by a motion, an encouragement to repeat a given rehabilitation activity, and/or terminates a given rehabilitation session.

According to the method of the invention, basing on the analysis of the emotional and/or physical condition of the patient, another personalized hologram is generated.

If the personalized hologram is a person or a being, then by reconverting and parametrizing a photograph and/or an image and/or a movie, the personalized hologram obtains gestures, facial expressions, and voice of this person or being.

The method according to the invention, additionally comprises loading a material in form of photographs, images, recorded movie, obtained from a loved one of the patient, and reconverting it into a personalized hologram. The personalized hologram is displayed in any suitable sizes.

During the rehabilitation session, feedback information is communicated, in real time, between the personalized hologram and the patient.

Motion capturing means and/or means for recording record the motion, gesture, emotions, and the engagement of the patient in performing the rehabilitation activity.

The means for generating a voice signal is a speaker, microphone, or the personalized hologram itself is a direct tool for communication with the patient.

Furthermore, the subject matter of the invention is a holographic device for rehabilitation of individuals with motor/neurological disabilities and/or motor/intellectual disabilities, including at least one of:
- a communication establishing means for establishing a communication with the receiving module in the central system for receiving a personalized hologram that is a three-dimensional image of an event, a landscape, a person, an architecture, individuals in a group, that induces emotional associations for a patient, and, optionally, that is related to the history of the patient,
- a receiving means for receiving a personalized hologram for receiving a personalized hologram from the central system onto the holographic device,
- an image displaying means for displaying a personalized hologram that by a motion and/or voice command issues a command for the patient to perform a rehabilitation activity,
- a signal generating means for generating a signal, to be communicated by the personalized hologram as a voice command, of instructions and/or recommendations to the patient to perform the rehabilitation activity,
- a capturing means for capturing motions, gestures, emotions, and engagement of the patient in performing the rehabilitation activity,
- a footage communicating means for communicating, to the receiving module in the central system, a footage of the rehabilitation activity performed by the patient, his/her emotional condition and the course of the rehabilitation session.

Preferably the means for generating a voice signal is speaker, microphone, or the personalized hologram itself is a direct tool for communication with the patient.

Also, the subject matter of the invention is a system for rehabilitation of individuals with motor/neurological disabilities and/or motor/intellectual disabilities, wherein:
a) a central system comprises:
   - a memory module for storing a patient identifier, basing on which a given rehabilitation session is initiated, and for storing data related to the rehabilitation session for the given patient,
   - a processor for converting and parametrizing a material in form of photographs, images, recorded movie, obtained from a loved one of the patient, into a personalized hologram that is a three-dimensional image of an event, landscape, person, architecture, individuals in a group, that induces emotional associations for a patient, and, optionally, that is related to the history of the patient, and for investigating, in real time, reactions of the patient to the personalized hologram, and for issuing current commands during an ongoing rehabilitation session, and for generating a report from the rehabilitation session,
   - a transmission module for transmitting the personalized hologram to the holographic device,
   - a receiving module for receiving, in real time, the footage of the rehabilitation session with the personalized hologram from the holographic device,
b) a holographic device comprising, at least one of:
   - a communication establishing means for establishing a communication with the receiving module in the central system for receiving a personalized hologram that is a three-dimensional image of an event, landscape, person, architecture, individuals in a group, that induces emotional associations for a patient, and, optionally, that is related to the history of the patient,
   - receiving means for receiving a personalized hologram from the central system onto the holographic device,
   - image displaying means for displaying a personalized hologram that, by a motion and/or voice command, issues a command for the patient to perform a rehabilitation activity,
   - a means for generating a signal, to be communicated by the personalized hologram as a voice command, of instructions and/or recommendations to the patient to perform the rehabilitation activity,
   - a capturing means for capturing motions, gestures, emotions, and engagement of the patient in performing the rehabilitation activity,
   - a footage communicating means for communicating, to the receiving module in the central system, a footage of the rehabilitation activity performed by the patient, his/her emotional condition, and the course of the rehabilitation session, and
c) optionally, a therapist's device and a server.

In the rehabilitation system according to the invention, the central system is integrated with the holographic device, or is a separate device.

Also, the subject matter of the invention is the use of the method according to the invention for stimulating, using a holographic device and/or above described system.

Preferably, the method for rehabilitation of individuals with motor/neurological disabilities and/or motor/intellectual disabilities using a holographic device, is characterized in that the image displaying means of a holographic device displays, during a single rehabilitation session, at least one personalized hologram that issues, in real time, by at least one means for generating a signal, a voice command and/or by a motion, a command to perform at least one rehabilitation activity by the patient before this holographic device, and at least one motion capturing means records the performance of said rehabilitation activity, and communicates the footage of a rehabilitation session, via a footage communicating means, to the central system to verify the correctness of performing the rehabilitation activity, where after the central system investigates, in real time, data obtained from the holographic device related to the performance of said rehabilitation activity by the patient and communicates, to the holographic device, commands that are then issued to the patient by the personalized hologram via at least one means for generating a signal as a voice command and/or by a motion, the personalized hologram being a three-dimensional image of an event, landscape, person, architecture, individuals in a group, that induces emotional associations for a patient.

Preferably, the holographic device according to the present invention is a device for patients with neurological and intellectual disabilities, who are relatively in good physical condition but it is very difficult if not impossible to run an efficient rehabilitation/medical therapy without establishing an appropriate relation with them. The holographic device operates basing on:
(i) loading materials (photographs of people, photographs of events, family recordings, movies, images, concerts, music, materials recorded on an ongoing basis) obtained from the family of the patient to parametrize the hologram appropriately for sessions with a particular patient,
(ii) recording every session of the patient with the holographic device,
(iii) analyzing performed sessions and introducing still new cognitive elements for the patient (a new person, new event, new photo, recording, movie, image etc),
(iv) analyzing progress of the patient and proceeding to sessions that are still more rich in people, images, actions,
(v) engaging of the loved ones of the patient of the present time into common sessions with the holographic device and introducing successively still newer and newer present elements and events (for individuals with intellectual delays - introducing socializing elements, possibilities of functioning in the modern world etc.).

For children with sensory integration dysfunctions or with intellectual disability it is important to establish the contact and to inspire trust in the child. Using the holographic device one can display best-loved characters, for example an animal, of the patient, in order to motivate the patient to perform appropriate tasks as a game.

In case of rehabilitating older individuals with Alzheimer's disease, who have problems with remembering new things but remember excellently events from the past, the personalized hologram may present persons from the past: acquaintances, somebody loved-one, a friend, an idol. The choice of the selected person, being a loved one for the patient, is to motivate him/her to do exercises thus establishing a relationship with the patient and to inspire trust in him/her.

The term "rehabilitation session", according to the invention, is meant to be an individualized rehabilitation proposed for the given patient. The rehabilitation session undergoes many phases, depending on the engagement of the patient, his/her dysfunctions, etc. Under the term of "rehabilitation session":
a. in the first phase of the rehabilitation, in the case of individuals with mental defect, autism, significant intellectual disability - it is meant to run a rehabilitation session displaying, using a holographic device, a personalized hologram showing a person, the patient has trust in, likes/loves or accepts, does not cry, does not exhibit aggression or escape. In this phase, the personalized hologram tries to establish a contact with the patient in order to inspire his/her trust,
b. in the first phase of the rehabilitation, in the case of individuals with the Alzheimer's disease - it is meant to run a rehabilitation session displaying, using a holographic device, a personalized hologram showing loved ones, known to the patient in the far past, such as the childhood, with which the patient establishes the contact more willingly than with the loved ones known in the present the latter being, frequently, not recognized nor associated by the patient. In this phase, the personalized hologram tries to establish a contact with the patient by means of general questions, such as *Do you remember the food we ate in the country*/*during holidays in childhood?* The personalized hologram attempts to provoke the patient to think back and associate,
c. in the second phase of the rehabilitation - it is meant to run a rehabilitation session displaying, using a holographic device, a personalized hologram showing proposals of some exercises and repetitions in the range of intellectual rehabilitation - the personalized hologram does not get nervous, irritated, frustrated or resigned as a human does and, despite many failures, again and again attempts to cooperate with the patient,
d. in the third phase of the rehabilitation - it is meant to run a rehabilitation session displaying, using a holographic device, a personalized hologram showing proposals of physical activities and exercises, encouraging the patient into common fun, dancing, painting - the holographic device has sensors/motion capturing means making it possible to evaluate, quantitatively and realistically, the engagement of the patient into the performed activities,
e. in the fourth phase of the rehabilitation - socializing phase - it is meant to run a rehabilitation session displaying, using a holographic device, a personalized hologram that cooperates with the patient, and his/her loved ones are engaged in the rehabilitation session, for example for individuals suffering from the Alzheimer's disease - persons of the present time - children, grandchildren, whereas in the case of individuals with mental defect, autism, significant intellectual disability, etc. - these will be for example parents, grandparents, etc.

Also, the term "rehabilitation session" performed using a holographic device relates, in the context of the present invention, to an activity, that needs to read out at least a part of the information relating to a given patient stored in the central system and/or other databases.

In turn, the term "rehabilitation activity" is understood as every activity to be done by the patient during a rehabilitation session - irrespective of the phase, out of the above described, to which this rehabilitation activity belongs. Under this term it is meant, for example, the initial acquainting with the personalized hologram, conversations related to the past, as well as for example painting, dancing, jumping, physical exercises, but also joint exercises and conversations between the patient, personalized hologram and a loved one. Also, the rehabilitation activity means, for example, a game of finding missing elements in a puzzle. Generally, it covers all the activities the patient is asked to do during a rehabilitation session.

According to the invention, the term "a loved one" is understood as a member of a family, a friend, acquaintances, an idol, an authority, a caretaker, i.e., this is a person the patient has a bond of confidence.

According to the invention, the rehabilitation session is based on materials obtained from the loved ones of the patient. Such materials may have a form of photographic documentation, images, music, recorded movies. It is preferable that the photographs have a field of depth, a perspective, such that one can create a personalized hologram basing on these photographs. It is also possible, when having no other materials, to use so called flat photographs. Computer software, according to the invention, converts and adjusts the photographs and the images, gives motion, facial expressions, and voice, creating "a personalized hologram". Thus, the "personalized hologram" is a three-dimensional image of an event, landscape, person, architecture, individuals in a group, inducing emotional associations for the patient. Optionally, the personalized hologram is related to the past, where the past may be, for example, an event that took place a couple of minutes ago. So, according to the present invention, the personalized hologram inducing an "emotional association" is based on showing an image that is known to the patient from the past, the patient being emotionally bond to this image. For example, it may be a friend, a lodge in a recreational land, or images of the best holidays in the patient's life.

Moreover, in the present invention, a new cognitive element for the patient means to show, by the personalized hologram, so far not yet displayed, during the given rehabilitation session, person, event, photograph, movie, image - this is a so called new person, new event, new photograph, new recording, movie, image, etc.

According to the invention, it is possible to issue commands - an example of performing an exercise, rehabilitation activity, etc., by the personalized hologram in real time. The feedback information in real time may be based on, for example, a formula calculated using data about the motion of the patient. Examples of data types that may be used for calculating the types of the feedback include: data about an instant position, data about the motion calculated basing on differences of positions, or data related to a comparison between an ideal model with an actual, exhibited by the patient, performance of an exercise, a motion, or a rehabilitation activity. The feedback information in real time may be communicated by the personalized hologram or displayed to the patient visually on a display or by means of audio signals.

The holographic device will be used for rehabilitating patients with motor, neurological, and intellectual disabilities, and the device will make it possible:
1) to provide patient, calm, steady and stressless work with a patient to an extent the patient wishes to do, i.e. depending on the quality and engagement of the patient into the conversation, the hologram's working time will be extended,
2) to develop communication skills, including communications with the loved ones in the present,
3) to develop cognitive education and socializing (in the case of individuals with intellectual delays),
4) to recall events, images and situations in order to pass from the far past to the present (in the case of individuals with Alzheimer's disease).

The present invention will be more fully understandable and appreciated basing on the following detailed description along with an attached drawing, in which:
- Fig. 1: shows a method for rehabilitation using a holographic device in one embodiment of the present invention,
- Fig. 2: shows a method for rehabilitation using a holographic device in a second embodiment of the present invention,
- Fig. 3: shows a system for rehabilitation using a holographic device in one embodiment of the present invention,
- Fig. 4: shows a holographic device in one embodiment of the present invention.
- Fig. 5: shows a holographic device in a second embodiment of the present invention.

### Detailed description

The invention is illustrated by the following non-limiting embodiment.

In the drawing, system 300 is shown for implementing a method of motor or neurological rehabilitation of individuals with motor/neurological or motor/intellectual disabilities. This system makes it possible to identify a patient of a rehabilitation session, to load or to call a personalized graphic material for a given patient, to convert the graphic material into a hologram or another 3D image, to start a new rehabilitation session or to continue an already initiated one, to analyze results of one or more sessions, to propose further steps (phases of rehabilitation), or methods of rehabilitation sessions. All of this to is possible within a single system for implementing a method of motor or neurological rehabilitation of individuals with motor/neurological or motor/intellectual disabilities. The system enables the therapist's work with the patient to be very elastic, making it possible to display personalized holograms referring to places, people, and/or events that are known to the patient.

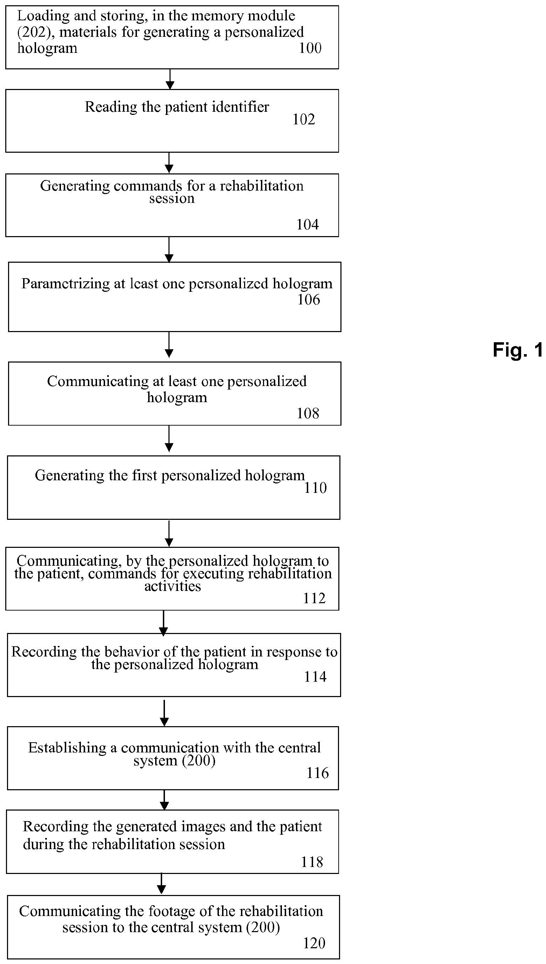

The method for rehabilitation of individuals with motor/neurological disabilities and/or motor/intellectual disabilities, in one embodiment shown in Fig. 1, comprises a holographic device 250, a central system 200, and, optionally, a therapist's device 212, or by the therapist's device 212, for example a mobile telephone, tablet or another mobile or stationary device. A memory module 202 of the central system 200 stores materials needed to create a personalized hologram. A folder in the memory module 202 is created for a new patient, in which the whole documentation is kept, obtained from loved ones of the patient. First of all, this is a medical history chart of the patient including his/her foregoing disease history, and also a documentation to be used during a rehabilitation session - photographs, images, music, recorded movies. Every patient obtains an individual identifier. It may be, be for example, any sequence of digits, name and surname, date of birth, etc. In order to initiate a rehabilitation session, one enters, 102, the patient identifier via an interface 210. The rehabilitation session may be initiated by introducing the identifier in a keyboard, 210, of the central system 200, or via a therapist's device 212. Then, commands for a given rehabilitation session are generated, 104. In this step, the therapist obtains a report of the whole foregoing therapy, as well as his/her recommendations after the last rehabilitation session. Indicated are recommended images to be displayed during the current rehabilitation session. The therapist selects an image to be the first personalized hologram during a given rehabilitation session. After selecting the material to be the personalized hologram, a step of parametrizing, 106, is executed, for the first personalized hologram. Then, the first personalized hologram is communicated, 108, to the holographic device 250. The holographic device 250 generates, 110, via image displaying means 406, the first personalized hologram. The personalized hologram communicates, 112, to the patient, commands for executing rehabilitation activities, by a gesture, gesture and voice command or only a voice command. The holographic device 250, records, 114, the behavior of the patient in response to the first personalized hologram. The recording is understood such that the holographic device 250 records, via a capturing means 410 for capturing motion and a footage communicating means 412, every motion, gesture, emotions, engagement of the patient and the therapist, as well as, in some cases, the behavior of an associating person in the rehabilitation session. Then, a communication establishing means 402 of the holographic device 250, establishes a communication, 116, with the central system 200. Recording step, 118, executed, for recording images, behavior of the patient, his/her reactions, emotions generated during the rehabilitation session. The footage of the rehabilitation session is communicated, 120, to the central system 200. According to the invention, the communication between the personalized hologram and the patient, therapist, and associating person is in real time, hence, according to the invention, the personalized hologram, on an ongoing basis, reacts to emotions, words, gestures of the patient during a given rehabilitation session. The ongoing reacting of the personalized hologram means that the personalized hologram relates, by a gesture, a voice command, etc., to the emotional and/or physical condition of the patient.

Optionally, the holographic device 250, is a portable device the patient may have, for example, at home, and he/she may do exercises on his/her own before this device, and perform commands communicated by the personalized hologram. Therefore, a rehabilitation session is initiated by logging the patient to the central system 200 and downloading recommendations for the rehabilitation session on the holographic device 250.

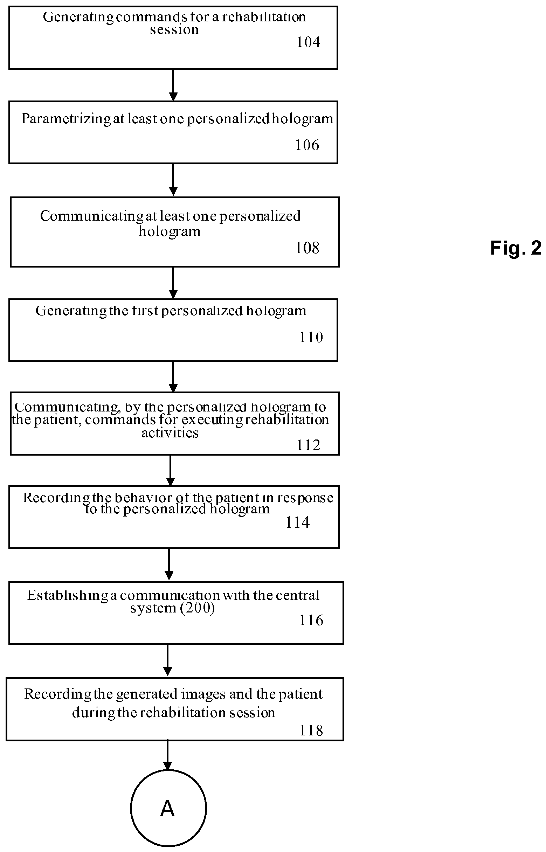

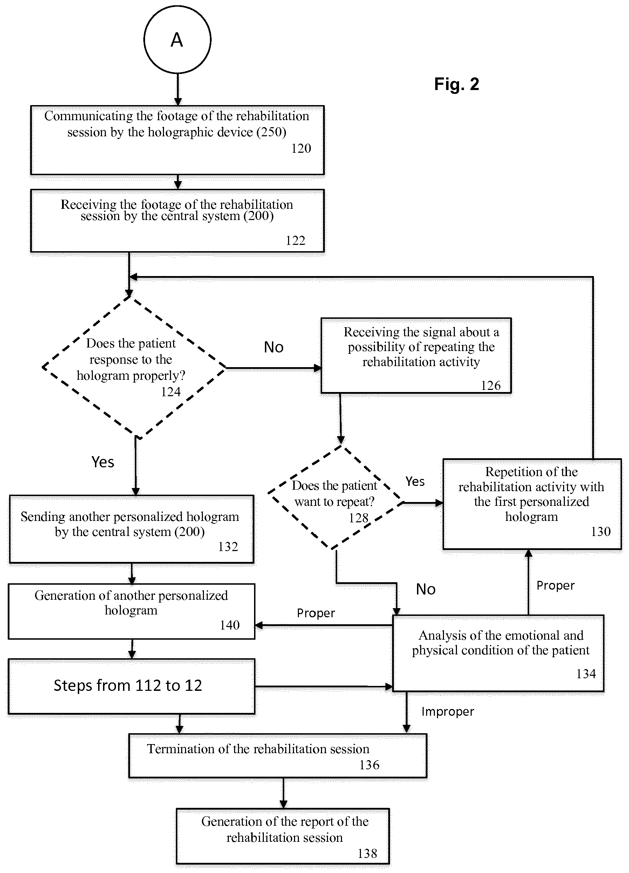

In an alternative embodiment of the method according to the invention, an example is shown in Fig. 2 wherein a patient also continues a therapy at home, and has already a patient identifier assigned. Therefore, in the first step, patient related data are read, 102, from the memory module, 202. Basing on the patient identifier, the interface, 210, for example a computer screen, displays the history of the disease and the rehabilitation of the patient, and the recently displayed personalized hologram. Then, commands are generated, 104, for a given rehabilitation session, and a material is selected to be displayed. The material is then parametrized, 106, into a personalized hologram. Then, this personalized hologram is communicated, 108, to the holographic device 250. The holographic device 250 generates, 110, via the image displaying means 406, the obtained personalized hologram. The personalized hologram communicates, 112, to the patient commands to execute rehabilitation activities by a gesture, a gesture and a voice command, or only a voice command. A conversation is possible between the patient and the personalized hologram. The holographic device 250 records, 114, the reactions of the patient to the displayed personalized hologram. Then, the holographic device 250 establishes a communication, 116, with the central system 200. The images, behavior of the patient, his/her reactions, emotions, etc., generated during the rehabilitation session, are recorded, 118. The footage of the rehabilitation session is communicated, 120, to the central system 200, and it is stored, 122, in the memory module 202 of the central system 200. Then, a verification step is executed, 124, to verify if the patient reacts appropriately to the displayed personalized hologram. In this step, for example, it is verified if the patient recognizes the personalized hologram - in the first phase of the rehabilitation, or if he/she establishes a contact with the personalized hologram, if he/she does the physical exercises or makes paintings, in the subsequent phase of the rehabilitation. The central system 200 obtains, in real time, a footage of the rehabilitation activity performed by the patient, and verifies, 124, the correctness of the patient reaction to the commands and instructions issued by the personalized hologram. In the case, when the capturing means 410 for capturing motions, or footage communicating means 412, do not register any expected gesture or motion, this information is sent, 126, to the central system 200 that, then, establishes a communication with the holographic device 250, and, via the means 408, for communicating voice and/or by a motion, the personalized hologram communicates, 128, an encouragement for the patient to repeat the rehabilitation activity. If the patient wishes to repeat and repeats, 130, the rehabilitation activity shown by the personalized hologram, again, a verifications step, 124, is executed to verify whether the rehabilitation activity is performed properly. If the rehabilitation activity is performed properly, then further rehabilitation activities are possible with the first or a subsequent personalized hologram, and the central system 200 plays, again, the first personalized hologram, or sends, 132, a subsequent personalized hologram. In turn, in the case when the patient does not wish to repeat, 128, the rehabilitation activity, then the central system 200 analyzes, 134, the recordings of gestures of the patient, his/her engagement and physical conditions obtained from the holographic device 250. In the case, the emotional and/or physical condition of the patient does not allow to continue the rehabilitation session, the rehabilitation session is terminated, 136. In turn, if the analysis, 134, of the emotional and/or physical condition is correct, then the first personalized hologram encourages, by a gesture, gesture and voice command, or only a voice command, the patient to perform yet another trial of the rehabilitation activity, and to continue to participate in the rehabilitation session actively. Also, it is possible that the patient does not wish to repeat, 130, the rehabilitation activity with the first personalized hologram, but his/her emotional and/or physical condition allows to continue the rehabilitation session. Then, the holographic device 250 generates, 140, depending on of the emotional and/or physical condition of the patient, via the image displaying means 406, another personalized hologram. Also, after appropriate performing the rehabilitation activity from the first personalized hologram, and if there were such commands for a given rehabilitation session, the holographic device 250 generates, 140, another personalized hologram. The personalized hologram communicates, 112, to the patient, a command to perform rehabilitation activities. Also, it is possible that the personalized hologram shows, on an ongoing basis, rehabilitation activities that are to be performed by the patient. The holographic device 250 records, 114, the behavior of the patient in response to the displayed personalized hologram. The term recording means that the holographic device 250, records, via capturing means 410 for capturing motion and footage communicating means 412 every motion, gesture, emotions, engagement of the patient and the therapist, as well as in some cases behavior of an associating person During a rehabilitation session. Then communication establishing means 402 of a holographic device 250, establishes a communication, 116, with the central system 200. Then, one executes a step, 118, of recording images, behavior of the patient, his/her reactions, emotions, etc., generated during the rehabilitation session. The footage of the rehabilitation session is communicated, 120, to the central system 200. The central system 200 obtains, 122, from the holographic device, the course of the current rehabilitation session of the patient. One or more personalized holograms may be displayed to the patient during a single rehabilitation session. The emotional and/or physical condition is checked by the central system 200 continuously in real time. After terminating, 136, the rehabilitation session, a report is generated, 138, of the rehabilitation session. The footage of the rehabilitation session is stored in the memory module 202 of the central system 200, and then it is analyzed by therapists in terms of continuing, changing, and/or developing the material communicated to the patient by the personalized hologram.

According to the method of the invention, the personalized hologram communicates with the patient in real time. It communicates commands, shows the appropriate performance of rehabilitation activities, encourages to further work, as well as it does reward in word for the appropriate performance of a rehabilitation activity. Also, it is possible, according to the following embodiment, that the personalized hologram shows a family movie while the therapist runs the rehabilitation session. Additionally, it is preferable that, during first rehabilitation sessions, the therapist observes the patient and his/her reactions to the personalized hologram displayed by the holographic device 250.

Also, it is possible, according to the invention, that the personalized hologram itself is a communication tool for communicating with the patient.

Optionally, during a rehabilitation session, the therapist may, via a therapist's device 216, communicate, to the central system 200, current recommendations related to given rehabilitation session and may change the chronology of displaying the personalized hologram and the commands it is to communicate to the patient. Also, the therapist may, via the therapist's device 216 or via the interface 210 of the central system 200, disable the voice signal of the personalized hologram while leaving the visual image only.

In an alternative embodiment of a method for rehabilitation of individuals with motor/neurological disabilities and/or motor/intellectual disabilities using a holographic device 250, the image displaying means 406, 506 displays, during a single rehabilitation session, at least one personalized hologram. The personalized hologram communicates, in real time, by at least one means for generating a signal, a voice command and/or by a motion, a command to perform at least one rehabilitation activity by the patient before the holographic device. The performance of a given rehabilitation activity is recorded by at least one capturing means 410, 510 for capturing motion and communicates the footage of the rehabilitation session, via a footage communicating means, 412, 512, to the central system 200 to verify the correctness of performing the rehabilitation activity. The central system 200 analyzes, in real time, the data obtained from the holographic device 250 and related to the performance of said rehabilitation activity by the patient, and communicates, to the holographic device 250, commands that are then communicated to the patient by the personalized hologram via at least one signal generating means 408, 508 for generating a signal, such as a voice command, and/or by a motion, the personalized hologram being a three-dimensional image of an event, landscape, person, architecture, individuals in a group, that induces emotional associations for a patient.

Additionally, if the processor 206 reading, in real time, a footage of the rehabilitation session communicated by the motion capturing means 410, 510 and/or the footage communicating means 412, 512, notices inappropriate performance of a rehabilitation activity done by the patient, then, basing on the given footage of the rehabilitation session, it verifies the emotional and/or physical condition of the patient and, in the case of any incorrectness in the emotional and/or physical condition of the patient, transmits a signal to the holographic device 250 to issue, by means of the personalized hologram displayed by the image displaying means 406, 506, and by means of a voice command, generated by a signal generating means 408, 508, and/or a motion, an encouragement to continue the rehabilitation activity and/or to repeat the rehabilitation activity, and/or terminates the rehabilitation session. The motion capturing means 410, 510 and/or the footage communicating means 412, 512 record the performed motion, gesture, emotions, and the engagement of the patient in performing the rehabilitation activity. The signal generating means 408, 508 for generating a voice signal is a speaker, a microphone, or the personalized hologram itself is a direct tool for communication with the patient.

The material in form of photographs, images, recorded movies, etc., is obtained from a loved one of the patient and is stored into the memory module 202 of the central system 200 whereafter the processor 206 reconverts and parametrizes it into a personalized hologram. In the case the personalized hologram is a person or a being, then, by reconverting and parametrizing a photograph and/or an image and/or a movie, the personalized hologram obtains gestures, facial expressions and voice of this person. Moreover, the image of an event, landscape, person, architecture, individuals in a group constituting the personalized hologram is related to the past of the patient.

During the rehabilitation session, feedback information is communicated, in real time, between the personalized hologram and the patient.

According to the present invention, the personalized hologram is displayed in any suitable sizes, which means that it may, for example, take a form of a human of realistic size, his/her whole silhouette, as well as the personalized hologram may be, for example, just a bust of a person. It is also possible, that the personalized hologram is played in a freely miniaturized form.

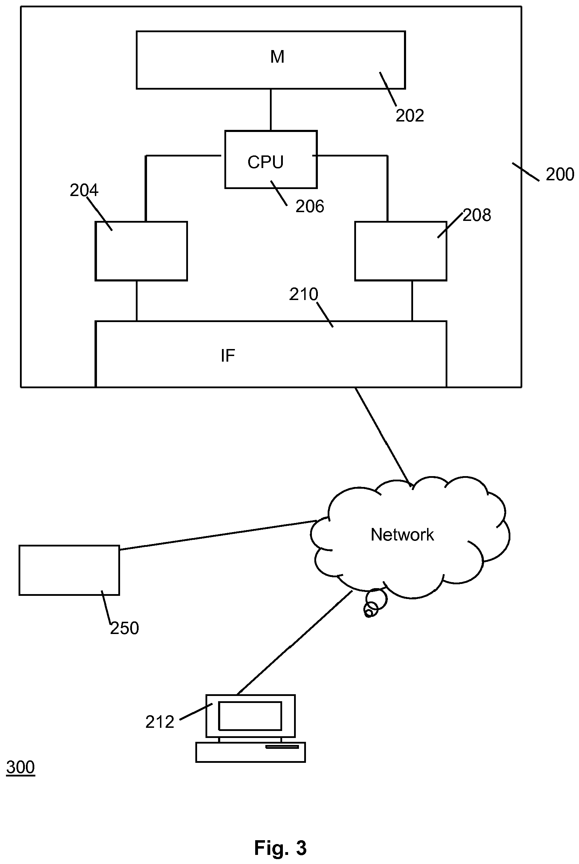

Fig. 3 shows one of many possible embodiments of a system 300 for implementing a method for rehabilitation according to the invention. This system is connected to a holographic device 250. The holographic device 250, for displaying images personalized for the given patient, is identified basing on unique address, for example MAC address (Media Access Control), ensuring the unique identification of the device. Communication of the holographic device 250 with the central system 200 may be executed by means of a telephone network. In such a case, the holographic device 250 is equipped with a modem, packet networks, mobile telephony networks or another type of network, that will allow for safe (encrypted) transmission of data from the holographic device 250 to the central system 200. The holographic device 250 is equipped with a storage media of small sizes, e.g., flash memory card, magnetic disks, such as internal hard disks and exchangeable disks, magnetooptical disks, and CD-ROM or DVD-ROM disks, etc.

The central system 200 has a computer software converting images from paper or digital form into a personalized hologram.

The system for implementing a method for rehabilitation 300 comprises a holographic device 250 with displaying means 406, 506 for displaying a personalized hologram, communication establishing means 402, 502 for establishing a communication with a central system 200, receiving means 404, 504 for receiving the personalized hologram, capturing means 410, 510 for capturing motion of the patient, and communicating means 412, 512 for communicating the footage of performed rehabilitation activities, and a central system 200.

In a memory module 202, being a part of the central system 200, identifiers/codes of individual patients are stored along with associated information. The memory module 202 has a data structure and a data registering unit. The data structure is adapted for receiving and storing captured data from the data registering unit and storing the preliminarily recorded data related to a rehabilitation session, reactions of a user of the rehabilitation session, etc. In this data structure, information is recorded related to the patient's diagnosis, patient's reaction to the rehabilitation session, patient's reaction to the first personalized hologram, as well as segregated and ordered folders of every patient, in which materials will be stored relating to him/her in form of a photographic documentation, images, music, materials obtained from the family and loved ones of the patient. This information is made available to the therapists and the family of the patient undergoing the rehabilitation session. In order to communicate with the holographic device 250, the central system 200 has a receiving module 204 for receiving information sent from the holographic device 250, about the rehabilitation session being in progress, about patient's emotions, his/her reactions to the rehabilitation session, etc. In one embodiment, it is a network Ethernet card. Other network technologies are also possible, however, they are not an aim of the present invention and therefore will not be discussed any more. Also, the central system 200 has a processor 206 adapted to associate the user's identifier with the personalized image and the information about the course of the rehabilitation session. The processor 206 is adapted to execute, automatically, an analysis and comparison of said captured data with preliminarily stored data, including a digitized audio library, and a visual course of every rehabilitation session. The processor 206 may play every session automatically and investigate differences in behavior of the patient during selected rehabilitation sessions, for example last five sessions, and display the changes and progress of patient's reaction to a given character of the personalized hologram. In different embodiments of the present invention, the processor 206 may be a microprocessor executing commands of computer software program, a circuit with a built-in firmware, or a microprocessor set. For the operation of the system it is important that the rehabilitation sessions are recorded properly and that one can check, for example, what rehabilitation sessions had been executed for a given patient, what was the patient's reaction to the personalized hologram, what is further diagnosis, what is the patient's progress during a rehabilitation session, how many further rehabilitation sessions are planned using selected personalized holograms, when the hologram should be exchanged to a subsequent personalized hologram, how many personalized holograms may be displayed during a single rehabilitation session. Also, the central system 200 has a transmission module 208 for transmitting information, for example the personalized hologram to the holographic device 250. In one embodiment, the same Ethernet network card is used as a transmission module 208. In an alternative embodiment, the system has an interface IF allowing to connect many devices for servicing the holographic device 250. The processor 206 records the course of the rehabilitation session within a data structure of the central system 200. Also, the processor 206 records information communicated by therapists after a rehabilitation session. The therapists communicate their comments and summaries of rehabilitation sessions to the central system 200, by means of any suitable electronic device 212, for example a telephone, a computer, etc. The therapists may communicate their comments via any suitable interface 210 of the central system 200.

Also, the data will be stored on an external server, whereby the memory module 202 will not be loaded.

The central system 200 makes available, to the therapists device 212, data about the course of the rehabilitation session of the patient stored in the data structure of the central system 200. Having so broad data about the patient's behavior during the rehabilitation session, the therapist may use it for analytical processes.

The processor 206 may have, e.g., a central processing unit (CPU), a graphics processing unit (GPU), a hardware processor core, and software for converting flat or digital photographs or other materials into a personalized form of the hologram or any combination thereof. Moreover, the central system 200 may include an interface 210, for example it may be a displaying unit (screen), an alphanumerical input device (e.g., a keyboard) and a pointing device of user interface (e.g., a mouse).

The holographic device 250 may have a receiving means 404, 504, for receiving a personalized hologram (e.g., control unit), a signal generating means 408, 508 for generating a signal (e.g., speaker), at least one capturing means 410, 510, for capturing patient's motion, patient's gestures (a sensor of the Global Positioning System (GPS)).

Moreover, the commands and the personalized hologram may be communicated or received by the holographic device 250, via a communication network using transmission medium by means of a communication establishing means 402, 502 using anyone of many transfer protocols (e.g., the Internet Protocol (IP), the Transmission Control Protocol (TCP), etc.). Exemplary communication networks may include a local network (LAN), a wide area network (WAN), packet data network (e.g., Internet), mobile telephony networks, wireless data networks, etc.

The communication establishing means 402 of the holographic device 250, with a receiver of the central system 200, may have a lot of antennas for wireless communication by means of at least one of: a single input- multiple outputs, multiple inputs-multiple outputs, or multiple inputs-single output. The communicating means 412, 512 for communicating the footage of the performed activities and the receiving means 404, 504 for receiving a personalized hologram include any media that are able to store, encode, or transfer commands to be performed by the holographic device 250, and also include digital or analog communication signals or other media, to facilitate communication of the central system 200 with the holographic device 250.

According to the invention, a system for rehabilitation of individuals with motor/neurological disabilities and/or motor/intellectual disabilities, comprises:
a) a central system 200, comprising:
   - a memory module 202 for storing a patient identifier, basing on which a given rehabilitation session is initiated, and for storing data related to the rehabilitation session for the given patient,
   - a processor 206 for converting and parametrizing a material in form of photographs, images, recorded movies, etc., obtained from a loved one of the patient, into a personalized hologram that is a three-dimensional image of an event, landscape, person, architecture, individuals in a group, that induces emotional associations for a patient, and, optionally, that is related to the history of the patient, and for investigating, in real time, reactions of the patient to the personalized hologram, and for issuing current commands during an ongoing rehabilitation session, and for generating a report from the rehabilitation session,
   - a transmission module 208 for transmitting the personalized hologram to the holographic device 250,
   - a receiving module 204 for receiving, from the holographic device 250, the footage of the rehabilitation session with the personalized hologram in real time,
b) holographic device 250, comprising, at least one of:
   - a communication establishing means 402, 502 with the receiving module 204 in the central system 200, for receiving the personalized hologram that is a three-dimensional image of an event, landscape, person, architecture, individuals in a group, that induces emotional associations for a patient, and, optionally, that is related to the history of the patient,
   - a receiving means 404, 504 for receiving the personalized hologram from the central system 200 onto the holographic device 250,
   - an image displaying means 406, 506 for displaying the personalized hologram, that, by a motion and/or voice command, issues a command for the patient to perform a rehabilitation activity,
   - a signal generating means 408, 508 for generating a signal, to be communicated by the personalized hologram as a voice command, of instructions and/or recommendations to the patient to perform the rehabilitation activity,
   - a capturing means 410, 510 for capturing motions, gesture, emotions, and engagement of the patient in performing the rehabilitation activity,
   - a footage communicating means 412, 512 for communicating, to the receiving module 204 of the central system 200, a footage of the rehabilitation activity performed by the patient, his/her emotional condition and of the course of the rehabilitation session, and
c) optionally, a therapist's device 212, and a server.

According to the present invention, the central system 200 is integrated with the holographic device 250, or is a separate device.

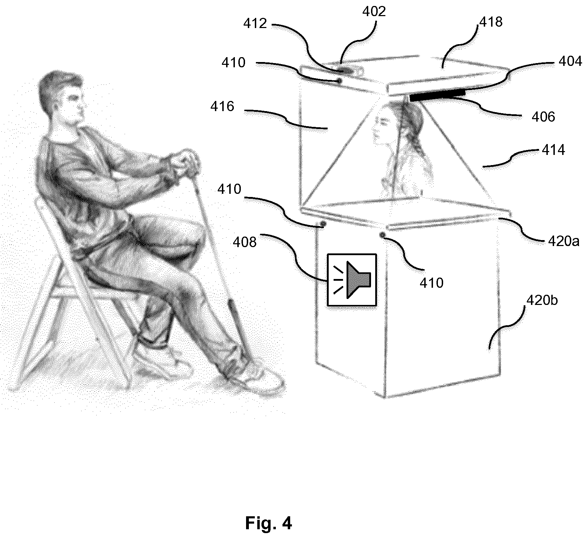

Fig. 4 shows a holographic device 250 according to a first embodiment. For example, the holographic device 250 has a form of a cuboid in which the rear wall 414 and the front wall 416, are glazed. The front wall 416 is situated at an appropriate angle to a flat base 420a, in terms between 4° and 85°. The basis is composed of a flat base 420a and a stable base 420b, located beneath the first one, in any form, for example a cuboid, cylinder, etc. Preferably, the side walls 422, 424 of the cuboid are shadowed, for example in black, in order to emphasize the displayed color of the personalized hologram. It is also possible that the side walls 422, 424 are made from a plastic material. In the space of the cuboid a hologram is displayed, a 3D image, by means of a projector 406 located in the upper part 418 of a holographic device 250. Also, it is possible to locate the projector 406 within the base 420a of the holographic device 250. The central system 200 transmits, via the transmission module 208, the personalized hologram to the holographic device 250, the hologram being received by a receiving means 404 for receiving the personalized hologram, that is then displayed by a displaying means 406 for displaying the hologram. The receiving means 404 for receiving a personalized hologram is located next to the projector 406 or it is integrated with it.

The holographic device 250 comprises at least one motion capturing means 410. In the case of a holographic device 250 shown in Fig. 4, the device has three capturing means 410. The capturing means 410 are intended for determining depth and distance, include location sensors (e.g., IR sensors, ultrasound sensors, GPS or like), etc., and may be freely distributed over the whole surface of the holographic device 250, both in its basis, as well as the upper part of the device. Also, the footage communicating means 412 record the quality and engagement of the patient into performed physical exercises or other rehabilitation activities. Also, the footage communicating means 412 transmit, on an ongoing basis, the recording of the rehabilitation session to the receiving module 204. The footage communicating means 412 may be a camera, a sensor or a similar element for recording a posture or emotions. Also, it is possible to use the so called kinect device comprising sensors and a camera for recording a rehabilitation session. The footage communicating means 412 is located in the upper part of the holographic device 250 along with the communication establishing means 402 for establishing a communication with the receiving module 204 of the central system 200. The holographic device 250 captures gestures, a gesture and voice command, or only a voice command. In order to communicate with the personalized hologram, it is possible to use signal generating means 408, generating voice, i.e., a microphone or speakers. The signal generating means 408 generating voice may be located both in the stable base 420b of the holographic device as well as in the upper part 418 of the holographic device.

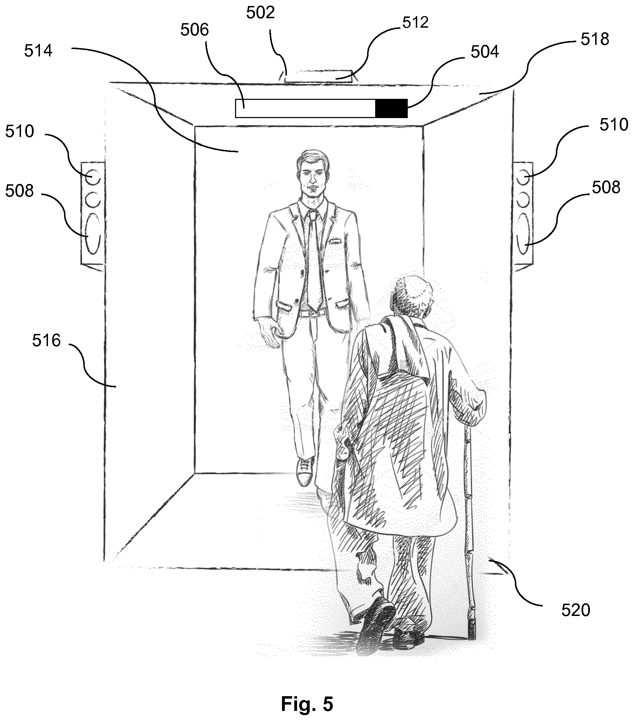

Fig. 5 shows a holographic device 250 according to a second embodiment. For example, the holographic device 250 has a form of a cuboid, in which the front wall 516 and the rear wall 514 are glazed. The side walls of the holographic device 250 may be shadowed or made from a nontransparent plastic material. The front wall 516 is situated at an appropriate angle relative to the base 520, the angle being between 4° and 85°. In the space of the cuboid, a hologram is displayed, a 3D image, by means of a projector 506 located in the upper part 518 of the holographic device 250. Also, it is possible to locate the projector 506 in the base 520 of the holographic device 250. The holographic device 250 is of a size making it possible to display the personalized hologram in realistic human sizes. Also, it is important that the personalized hologram plays the person, a hologram of which it is, as well as possible. It is possible to modify the brightness level of the personalized hologram before displaying it. The modification of the brightness level of the personalized hologram may include modifying the brightness or sharpening the facial features of the personalized holograms. In the holographic device 250 according to this embodiment, a signal generating means 508 for generating a signal and a capturing means 510 for capturing motions, are located on the side walls of the holographic device. A footage communicating means 512 for communicating the footage is located in the upper part of the holographic device 250 along with a communication establishing means 502 for establishing a communication with the receiving module 204 of the central system 200. Similarly, a means 506 for generating a personalized hologram is located along with a receiving means 504 for receiving the personalized hologram either within the base 520 of the holographic device or within its upper part 518.

The central system 200 communicates, via a transmission module 208, a personalized hologram to the holographic device 250, the hologram being received by the receiving means 404, 504 for receiving the personalized hologram that is then displayed by the displaying means 406, 506 for displaying the image, i.e., the personalized hologram. Also, by means of a projector 406, 506 it is possible to display traditional images, e.g., photographs.

According to the invention, it is also possible to use a plurality of holographic devices during a single rehabilitation session. For example, one personalized hologram will present, on the first holographic device, the motion to be performed by the patient, whereas a second personalized hologram, in a second holographic device, will stimulate and encourage the patient for further efforts.

### List of references

200 central system
202 memory module
204 receiving module
206 processor
208 transmission module
210 interface
212 therapist's device
250 holographic device
300 system for implementing a method for rehabilitation
402 communication establishing means for establishing a communication with a receiver of the central system (200)
404 receiving means for receiving a personalized hologram
406 image displaying means
408 signal generating means for generating a signal
410 capturing means for capturing rehabilitation activities of the patient;
412 footage communicating means
414 rear wall
416 front wall
418 upper part of a holographic device
420a flat base of a holographic device
420b stable base of a holographic device
422 rights side wall
424 left side wall
502 communication establishing means with a receiver of the central system (200)
504 receiving means for receiving a personalized hologram
506 image displaying means
508 signal generating means for generating a signal
510 capturing means for capturing the performance of the patient's rehabilitation activities
512 footage communicating means
514 rear wall
516 front wall
518 upper part of the holographic device
520 base of the holographic device

## Claims

1. A method for rehabilitation of individuals with motor/neurological disabilities and/or motor/intellectual disabilities, the method comprising:
- generating (104), in a central system (200), recommendations for a given rehabilitation session for a given patient identifier,
- parametrizing (106) at least one personalized hologram, this being a three-dimensional image of an event, landscape, person, architecture, individuals in a group, inducing emotional associations for a patient, and, optionally, related to the history of the patient,
- sending (108), by the central system (200), at least one personalized hologram to a holographic device (250),
- generating (110), by an image displaying means (406), of a holographic device (202), a first personalized hologram,
- issuing (112) to the patient, by the first personalized hologram, a command to perform rehabilitative actions by a motion and/or voice command,
- recording (114), by a capturing means (410), for capturing motions, and/or by a footage communicating means (412) a fact of performing, by the patient, the rehabilitation activities, his/her emotional condition, and the course of the rehabilitation session,
- Establishing a communication (116) with a receiving module (204) of the central system (200),
- recording (118) the generated images and the patient during a given rehabilitation session,
- communicating (120), in real time, the footage of a given rehabilitation session to the central system (200) in order to check the emotional and/or physical condition and/or to check the correctness of performing, by the patient, the rehabilitation activities.

2. The method for rehabilitation according to claim 1, wherein, additionally, the method comprises:
- introducing (102) a patient identifier into the central system (200),
- receiving (122), in real time, by the holographic device (250), information from the central system (200) about the course of the rehabilitation session,
- verification (124), in real time, of the course of the rehabilitation session and the correctness of the performed rehabilitation activities.

3. The method for rehabilitation according to anyone of the above claims, wherein in the case of a negative result of the verification (124) of performing rehabilitation activities by the patient during a given rehabilitation session, the method comprises receiving (130) a signal from the central system (200) about a possibility of repeating (126) the rehabilitation activity that is repeated (130) by the patient, and in the case the patient wishes not to repeat the rehabilitation activity, the central system (200) analyzes (134) the emotional and/or physical condition of the patient, and, basing on the read emotional and/or physical condition of the patient, communicates a signal to a holographic device (250), that, by means of a personalized hologram displayed by the image displaying means (406, 506), issues by means of a voice command, via a signal generating means (408, 508) for generating voice, and/or by a motion, an encouragement to repeat (130) a given rehabilitation activity, and/or terminates (136) a given rehabilitation session.

4. The method for rehabilitation according to claim 3, wherein basing on the analysis (134) of the emotional and/or physical condition of the patient, the method generates (140) yet another personalized hologram.

5. The method for rehabilitation according to claim 1, wherein in the case the personalized hologram is a person or a being, then by reconverting and parametrizing a photograph and/or an image and/or a movie, the personalized hologram obtains gestures, facial expressions, and voice of this person or being.

6. The method for rehabilitation according to anyone of the above claims, wherein, additionally, the method comprises loading (100) a material in form of photographs, images, recorded movies, etc., obtained from a loved one of the patient, and reconverting it into a personalized hologram.

7. The method for rehabilitation according to claim 1, wherein during a rehabilitation session, feedback information is communicated, in real time, between the personalized hologram and the patient.

8. The method for rehabilitation according to claim 1, wherein the personalized hologram is displayed in any suitable sizes.

9. The method for rehabilitation according to claim 1, wherein the motion capturing means (410, 510) and/or the footage communicating means (412, 512) record (114) the motion, gesture, emotions, engagement of the patient in performing the rehabilitation activity.

10. The method for rehabilitation according to claim 1, wherein the signal generating means (408, 508) generating a voice signal is a speaker, microphone, or the personalized hologram itself is a direct tool for communication with the patient.

11. A holographic device (250) for rehabilitation of individuals with motor/neurological disabilities and/or motor/intellectual disabilities including at least one of:
- a communication establishing means (402, 502) for establishing a communication with the receiving module (204) in the central system (200) for receiving a personalized hologram that is a three-dimensional image of an event, landscape, person, architecture, individuals in a group, that induces emotional associations for a patient, and, optionally, that is related to the history of the patient,
- a receiving means (404, 504) for receiving a personalized hologram from the central system (200) onto the holographic device (250),
- an image displaying means (406, 506) for displaying a personalized hologram that, by a motion and/or voice command, issues a command for the patient to perform a rehabilitation activity,
- a signal generating means (408, 508) for generating a signal, to be communicated by the personalized hologram as a voice command, of instructions and/or recommendations to the patient to perform the rehabilitation activity,
- a capturing means (410, 510) for capturing motions, gesture, emotions, and engagement of the patient in performing the rehabilitation activity,
- a footage communicating means (412, 512) for communicating, to the receiving module (204) in the central system (200), a footage of the rehabilitation activity performed by the patient, his/her emotional condition and the course of the rehabilitation session.

12. The holographic device (250) according to claim 11, wherein a signal generating means (408, 508) for generating a voice signal is a speaker, microphone, or the personalized hologram itself is a direct tool for communication with the patient.

13. A system for rehabilitation of individuals with motor/neurological disabilities and/or motor/intellectual disabilities, wherein:
a) a central system (200) comprises:
- a memory module (202) for storing a patient identifier, basing on which a given rehabilitation session is initiated, and for storing data related to the rehabilitation session for the given patient,
- a processor (206) for converting and parametrizing a material in form of photographs, images, recorded movies, etc., obtained from a loved one of the patient, into a personalized hologram that is a three-dimensional image of an event, landscape, person, architecture, individuals in a group, that induces emotional associations for a patient, and, optionally, that is related to the history of the patient, and for investigating, in real time, reactions of the patient to the personalized hologram, and for issuing current commands during an ongoing rehabilitation session, and for generating a report from the rehabilitation session,
- a transmission module (208) for transmitting the personalized hologram to the holographic device (250),
- a receiving module (204) for receiving, in real time, the footage of the rehabilitation session with the personalized hologram from the holographic device (250),
b) a holographic device (250) comprising, at least one of:
- a communication establishing means (402, 502) for establishing a communication with the receiving module (204) in the central system (200) for receiving a personalized hologram that is a three-dimensional image of an event, landscape, person, architecture, individuals in a group, that induces emotional associations for a patient, and, optionally, that is related to the history of the patient,
- a receiving means (404, 504) for receiving a personalized hologram from the central system (200) onto the holographic device (250),
- an image displaying means (406, 506) for displaying a personalized hologram that, by a motion and/or voice command, issues a command for the patient to perform a rehabilitation activity,
- a signal generating means (408, 508) for generating a signal, to be communicated by the personalized hologram as a voice command, of instructions and/or recommendations to the patient to perform the rehabilitation activity,
- a capturing means (410, 510) for capturing motions, gesture, emotions, engagement of the patient in performing the rehabilitation activity,
- a footage communicating means (412, 512) for communicating, to the receiving module (204) in the central system (200), a footage of the rehabilitation activity performed by the patient, his/her emotional condition and of the course of the rehabilitation session, and
c) optionally, a therapist's device (212) and a server.

14. The system of rehabilitation according to claim 10, wherein the central system (200) is integrated with the holographic device (250), or it is a separate device.

15. The use of a method defined in claims 1-10 for stimulating, using a holographic device (250) defined in claims 11-12 and/or of a system defined in claims 13-14.
